# NEW EUROPEAN PATENT SPECIFICATION

(11) **EP 1 575 469 B2**
(45) Date of publication and mention of the opposition decision: **18.12.2019**
(45) Mention of the grant of the patent: 14.11.2012
(21) Application number: 03734496.7
(22) Date of filing: 09.06.2003
(51) Int. Cl.: A61F 13/15

(54) **DISPOSABLE UNDERGARMENT WITH A GATHERED CROTCH MEMBER AND METHOD FOR THE MANUFACTURE THEREOF**
WEGWERFWINDEL MIT ZUSAMMENGEZOGENEM SCHRITTBEREICH UND HERSTELLUNGSVERFAHREN
SOUS-VETEMENT JETABLE DOTE D'UN ELEMENT DE FOURCHE RASSEMBLE ET SON PROCEDE DE FABRICATION

(30) Priority: 12.12.2002 US 318329
(43) Date of publication of application: 21.09.2005
(73) Proprietor: KIMBERLY-CLARK WORLDWIDE, INC., Neenah, WI 54956 (US)
(72) Inventor: THORSON, Russell, E., Appleton, WI 54914 (US); VAN GOMPEL, Paul, T., Hortonville, WI 54944 (US); GROSS, Jacqueline, A., Neenah, WI 54956 (US)
(74) Representative: Dehns
(86) International application number: PCT/US2003/018169
(87) International publication number: WO 2004/054396

(56) References cited:
- EP-A- 0 321 980
- EP-A- 0 321 985
- EP-A2- 1 249 214
- WO-A-02/24011
- WO-A1-93/17648
- GB-A- 2 263 859
- US-A- 4 655 760
- US-A- 4 747 846
- US-A- 4 846 827
- US-A- 5 376 198
- US-B1- 6 217 563
- DATABASE WPI Section Ch, Week 200012 Derwent Publications Ltd., London, GB; Class A35, AN 2000-129339 XP002293846 & JP 11 348221 A (IDEMITSU PETROCHEM CO LTD) 21 December 1999 (1999-12-21)

## Description

### BACKGROUND

The present invention relates generally to a method for the manufacture of a disposable undergarment having a gathered crotch member.

Disposable undergarments can be configured in many different forms. For example, disposable absorbent garments can be configured as a pant-type, pull-on garment, or as a diaper-type product that is drawn up between the legs and fastened about the waist with various fastening systems. In some configurations, the garment is formed from a crotch member attached to a body panel, which may be elongateable. Typically, the crotch member is secured to the body panel when the body panel is in a relaxed condition. In addition, the crotch member is often secured across the entire width of the portion of the crotch member that overlaps the body panel. As such, the crotch member, which is often non-elastic, can reduce or otherwise impede the elongation of the body panel, thereby reducing its ability to conform to the body of the user. In addition, the crotch member typically is not able to conform to the body of the user independently of the body panel, and can therefore distort the panels when fitted to a user.

US-5,376,198 describes a method for making a stretchable absorbent article having the features of the preamble of claim 1. EP 1249214 A2 discloses all of the features of claim 1 with the exception of "wherein said elongating said body panel material (4, 6) in said first direction to said elongated condition comprises elongating said body panel material (4, 6) between about 50% and about 250% in said first direction."

Therefore the need remains for an improved undergarment that conforms to the body of the user during use without interference from a crotch portion thereof, and for improved methods and assemblies for manufacturing such undergarments.

### SUMMARY

According to the invention, there is provided a method as claimed in claim 1.

The various presently preferred embodiments provide significant advantages over other methods for the manufacture and use thereof. For example, the gathered crotch member does not restrict the elongation of the body panel, but rather can be elongated therewith. Accordingly, as the body panel is applied to the user, the body panel material can elongate and conform to the body of the user, with the crotch member elongating with the body panel. This configuration provides for a body conforming fit of the body panel and also a snug fit of the crotch member, which is configured as an absorbent insert, to the body of the user.

The foregoing paragraphs have been provided by way of general introduction, and are not intended to limit the scope of the following claims. The presently preferred embodiments, together with further advantages, will be best understood by reference to the following detailed description taken in conjunction with the accompanying drawings.

### BRIEF DESCRIPTION OF THE DRAWINGS

Many of the features and dimensions portrayed in the drawings, and in particular the presentation of layer thicknesses and the like, have been somewhat exaggerated for the sake of illustration and clarity.
FIGURE 1 is a plan view of a first embodiment of an absorbent garment taken from the bodyside thereof.
FIGURE 2 is a schematic illustration of a cross-sectional view of the absorbent garment taken along line 2-2 in Figure 1.
FIGURE 3 is a schematic-illustration of a cross-sectional view of the absorbent garment taken along line 3-3 in Figure 1.
FIGURE 4 is a schematic illustration of a cross-sectional view of the absorbent garment taken along line 4-4 in Figure 1.
FIGURE 4A is a schematic illustration of a cross-sectional view of an alternative embodiment of the absorbent garment taken along line 4-4 in Figure 1.
FIGURE 4B is a schematic illustration of a cross-sectional view of an alternative embodiment of the absorbent garment taken along line 4-4 in Figure 1.
FIGURE 4C is a schematic illustration of a cross-sectional view of an alternative embodiment of the absorbent garment taken along line 4-4 in Figure 1.
FIG. 5 is a schematic illustration of one embodiment of manufacturing assembly used to assemble an undergarment.

### DETAILED DESCRIPTION OF THE PRESENTLY

### PREFERRED EMBODIMENTS

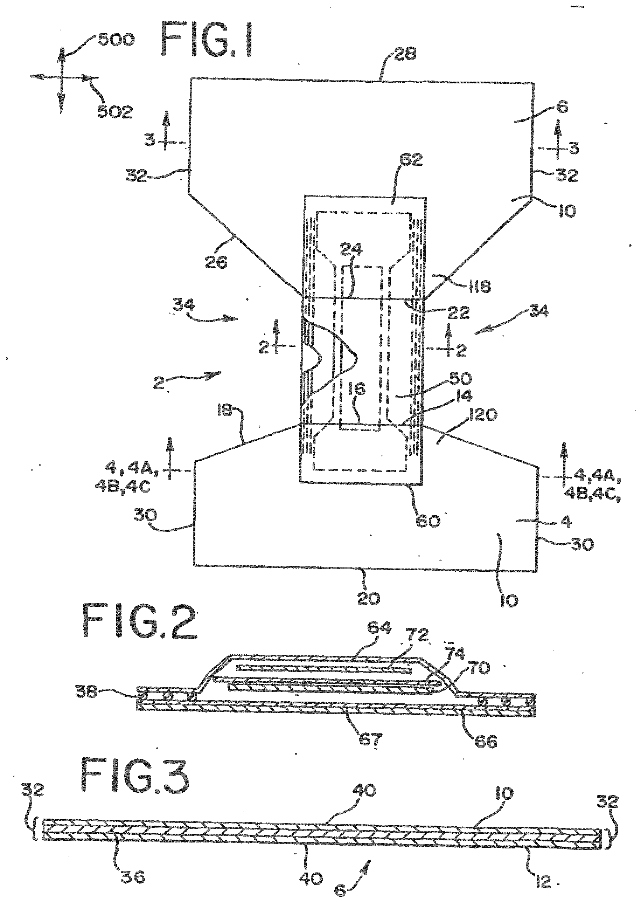

It should be understood that the term "longitudinal," as used herein, means of or relating to length or the lengthwise direction, for example the lengthwise direction **500** of the undergarment shown in FIG. 1. The term "laterally," as used herein means situated on, directed toward or running from side to side, for example the side to side direction **502** of the undergarment shown in FIG. 1. The term "first direction" generally refers to a path, line or course rather than a vector, and applies equally to opposite orientation along the path, line or course, including for example and without limitation movement along a path, line or course in both directions. Likewise, the term "second direction" generally refers to a path, line or course rather than a vector (not orientation dependent), and includes for example and without limitation movement along a path, line or course in both directions. In one example, the first direction is defined by and refers to one of the longitudinal and lateral directions, while the second direction refers to the other of the longitudinal and lateral directions.

The term "bodyside" should not be interpreted to mean in contact with the body of the user, but rather simply means the side that would face toward the body of the user, regardless of whether an undergarment is actually being worn by the user and regardless of whether there are or may be intervening layers between the component and the body of the user. Likewise, the term "garment side" should not be interpreted to mean in contact with the garments of the user, but rather simply means the side that faces away from the body of the user, and therefore toward any outer garments that may be worn by the user, regardless of whether the undergarment is actually being worn by a user, regardless of whether any such outer garments are actually worn and regardless of whether there may be intervening layers between the component and any outer garment.

The term "machine direction" means the direction of flow as the various members and webs progress along the fabrication line and process. It should be understood that various separate members or webs can each be traveling in a machine direction, but with the various machine directions not necessarily being parallel or oriented in the same direction. For example, a first component such as a web may be traveling a first machine direction, which is substantially perpendicular to the travel of another component, such as an absorbent insert, in a second machine direction.

The term "cross direction" means the direction substantially perpendicular to the machine direction.

The term "downstream" means that one item is positioned more closely to the output or finished product end of the machine and/or process relative to another item. Conversely, the term "upstream" means that an item is positioned more closely to the input end of the machine or process relative to another item. For example, the output end is downstream of the input end, and vice versa, the input end is upstream of the output end.

The phrases "removeably attached," "removeably attaching," "removeably connected," "removeably engaged," "releasably attached," "releasably connected," or "releasably engaged," and variations thereof, refers to two or more elements being connected or connectable such that the elements tend to remain connected absent a separation force applied to one, both or all of the elements, and where the elements are capable of being separated upon the application of a separation force. The required separation force is typically beyond that encountered while wearing the absorbent garment.

The phrases "fixedly secured," "fixedly engaged," "fixedly attached," "fixedly connected," and variations thereof, refers to two or more elements being connected or connectable such that they are not disconnected or otherwise separated, and are not intended to be separated or disconnected, during the normal operation and use of the absorbent garment.

The term "web" refers to a continuous stream of material, whether made from one or more layers or substrates, and regardless of whether it may have noncontinuous, discrete items disposed thereon.

The terms "connecting," "coupled," "attached," and "secured," and variations thereof, broadly covers two or more items being directly connected one to the other, or by way of one or more intervening members or components.

Referring to FIGS. 1-4, an undergarment **2** includes a first, front body panel **4** and a second, rear body panel **6**. The term "body panel" refers to the portion(s) of the undergarment, whether made of one or more layers or substrates or of one or more pieces or components, that is/are fitted circumferentially around at least the waist region of the user, including for example the user's lower back, buttocks, hips and abdomen. The first and second body panels each have an inner, bodyside surface **10** and an outer, garment side surface **12**. The first, front body panel **4** has a first edge **14** having a crotch portion **16** and a leg opening portion **18** and a second terminal edge **20** that is linear but can assume other shapes. Likewise, the second, rear body panel **6** has a first edge **22** having a crotch portion **24** and a leg opening portion **26** and a second terminal edge **28**, which is linear but can assume other shapes. Each of the first and second body panels have an outboard side edge **30**, **32** formed along the outer periphery of the opposite side portions of the first and second body panel. It should be understood that the outboard side edges of the front and rear body panels could have different lengths relative to each other.

In one embodiment, one or more, and preferably a plurality, meaning two or more, elastic elements are secured to each of the first and second body panels. In one embodiment, a plurality of elastic elements are spaced across substantially the entire waist portion of the front and rear body panel, although they may be spaced across a lesser length. For example, elastic elements can extend along the upper waist portion and along the lower terminal edge defining in part a leg opening.

In one embodiment, the front body panel has a "non-elasticized" area wherein there are no elastic elements, or other elastic or elastomeric backing members, incorporated therein or making up any portion of the thickness or cross-section of the body panel at that area. It should be understood, that in various embodiments, one or more separate waist bands, with or without elastic elements, can be secured to one or both of the rear and front body panels, for example along the upper terminal edges **20**, **28** thereof. Likewise, one or more separate leg bands can be secured to one or both of the rear and front body panels along the leg open portions **18**, **26** adjacent the leg openings **34**. Alternatively, one or both of the body panels can be formed without any elastic elements.

The various waist and leg elastic elements can be formed from rubber or other elastomeric materials. One suitable material is a LYCRA® elastic material. For example, the various elastic elements can be formed of LYCRA® XA Spandex 540, 740 or 940 decitex T-127 or T-128 elastics available from E.I. duPont De Nemours and Company, having an office in Wilmington, Delaware.

Referring to FIGS. 1, 3 and 4-4C, each body panel **4**, **6** is formed as a composite, or laminate material, otherwise referred to as substrates or laminates, with one or more elastic elements 36 sandwiched therebetween. The elastic element can be formed from a film, which can be apertured, ribbons, strands or other types of elastic elements. In one embodiment, two or more layers **40** are bonded to the elastic element **36**, and/or each other, with various adhesives, such as hot melt, or by other techniques, including for example and without limitation ultrasonic bonding and heat pressure sealing. In one embodiment, the two layers are made of a non-woven material such as a spunbond material, a bonded carded material or other known materials. It should be understood that the body panels can be comprised of more than two layers or substrates. Of course, it should be understood that other knitted or woven fabrics, non-woven fabrics, elastomeric materials, polymer films, laminates and the like can be used to form one or more of the body panel layers. The term "non-woven" web or material, as used herein, means a web having a structure of individual fibers or filaments that are interlaid, but not in an identifiable manner and without the aid of textile weaving or knitting, as in a knitted or woven fabric.

In various embodiments, the body panel material may be substantially permeable to air or substantially impermeable to air. The body panel material also may be substantially liquid-permeable or substantially liquid-impermeable. In particular arrangements, the body panel material may be substantially nonelastomeric. In other aspects, the body panels can include an elastomeric material that is elastomerically stretchable at least along one or both of the lateral article width and the longitudinal article length. Examples of such elastomeric composite materials can include a vertical filament laminate (VFL), neck-bonded-laminate (NBL), a strech-bonded-laminate (SBL), a necked-stretch bonded laminate (NSBL) or a necked-thermal laminate, or the like, as well as combinations thereof. Exemplary NBL, SBL, and NSBL materials are described in U.S. Patent Nos. 5,226,992, 4,981,747,4.965,122, 5,336,545, 5,385,775, 5,414,470, 4,720,415, 4,789,699, 4,781,966, 4,657,802, 4,652,487, 4,655,760, 5,116,b62 and 5,114,781. Exemplary WL materials are described in U.S. Provisional Patent Application Serial Number 60/204,307, filed May 15, 2000 and entitled "Method and Apparatus for Producing Laminated Articles," and PCT application WO 01/88245 A2, both assigned to Kimberly-Clark Worldwide, Inc., the Assignee of the present application, with the entire disclosures of both being hereby incorporated herein by reference. Such laminates can provide an improved combination of cloth-like feel and elastomeric stretchability. The body panels can be composed of materials that are elastic or elastomeric and exhibit biaxial stretch characteristics or MD/CD stretch characteristics, or that are extensible composites. Additional waist and leg elastic elements can be added to, but are not necessarily required by, the body panels.

As shown in FIG. 1, in one embodiment, the entirety of the body panels **4**, **6** are elasticized, such that the entire body panel conforms to the body of the user without any spacing between the body panel and the user's body, and without the attendant bulkiness of a non-elasticized material.

In one embodiment, the body panels are breathable, cloth-like, multidirectional nonwoven laminates with stretch and/or extensible properties. In one embodiment, the non-woven layers are pre-necked, for example between about 10% and about 80%, in the longitudinal direction, which provides extensibility in the longitudinal direction with minimum force.

The terms "extensible," "extensibility," and variations thereof as used herein means capable of being extended, and providing a selected elongation, for example between about 5% and about 70%, when subjected to an applied tensile force. The body panel also is capable of providing a selected, sustained deformation when subjected to an applied tensile force and then allowed to relax for a selected time period beginning immediately after removal of the tensile force. The sustained deformation is a substantially permanent deformation. The selected elongation and sustained deformation occur at least along the longitudinal direction of the garment, although it should be understood that it also could occur along the lateral direction, or both. Various extensible materials, and other acceptable materials that can be used for the body panels are described for example in U.S. Patent No. 6,217,563, issued April 17, 2001 to Kimberly-Clark Worldwide, Inc., the same Assignee as the present application.

The extensibility of the exemplary non-woven material provides an increase in surface area without the retractive force of elastomeric materials. In one embodiment, body panel is extensible in at least the cross-direction, or longitudinal direction, with the material providing an elongation of at least about 1 cm when subjected to a tensile force of 11.8 grams per cm. In addition, the body panel provides a substantially permanent deformation of at least about' 20% when it is subjected to a tensile force of 19.70 grams per cm and is then allowed to relax under a zero applied force for a period of 1 minute. Of course, it should be understood that the body panel can also be made extensible in the lateral direction.

In one embodiment, the front and rear body panels **4**, **6** are made of non-woven laminates of two layers of longitudinally extensible 0.60 osy polypropylene spunbond material with elongated strands of Lycra® elastic sandwiched between the spunbond layers and thereafter adhesively bonded. In particular, the body panel material is necked in the cross direction. As used herein, the term "necked," and variations thereof, refers to any material that has been constricted in at least one dimension by applying a tensioning force in a direction that is perpendicular to the desired direction of neck-down. Processes that may be used to constrict a material in such a manner include, for example and without limitation, drawing processes. The elastics are then elongated in the machine direction and secured to the body panel material. The elastics are then allowed to retract so as to gather the necked spunbond material in the machine direction (lateral body panel direction) thereby creating an elastically gathered non-woven body panel with longitudinal extensibility. The term "gather," and variations thereof, as used herein means puckered, or contracted into folds or wrinkles, which should be understood as including micro-pleats. In this way, the body panel can be elongated in both the longitudinal and lateral direction to conform to the body of the user when the garment is applied thereto. In particular, as the user pulls the garment up over their hips, the non-woven laminate body panels stretch in the lateral direction while the leg regions of the front and rear body panels conform to the crotch and body lines of the user. At the same time, the body panel material extends in the longitudinal direction to conform to the buttocks and stomach of the user. The extensibility of the body panels follows the natural curvature of user's body to provide conformance thereto. As the body panel extends in the longitudinal direction, the spacing between the laterally extending elastic elements, incorporated in one embodiment, will increase.

The body panel **4**, **6** non-woven material is substantially hydrophobic, which may optionally be treated with a surfactant or otherwise processed to impart a desired level of wettability and hydrophilicity. In one particular embodiment of the invention, the body panel is a nonwoven, wire-weave spunbond polypropylene fabric composed of about 1.6 denier fibers formed into a web having a basis weight of about 0.6 osy. One suitable non-woven material is the Corinth 0.60 osy, 1.6 dpf wireweave, nonwettable Metallocene (EXXON ACHIEVE 2854 PP) spunbond material manufactured by Kimberly-Clark Corporation, the assignee of the present application.

Referring to FIG. 1, a crotch member **50** connecting the front and rear body panels **4**, **6** can be folded such that the side edges **30**, **32** of the front and rear body panels **4**, **6** are aligned wherein they can be fixedly secured at a seam. The seam can be formed by bonding, sewing or otherwise attaching the side edges. Alternatively, the product can remain "open," wherein the body panels are releasably secured with one or more fastening members as explained below.

In one embodiment, the garment includes a combination of side edges that are secured to form a seam and fastening members that allow the fit of the undergarment to be adjusted. For example, in one embodiment, fastening members are attached to the front body panel and extend inboard relative to the outboard side edge **30** of the front body panel **4** from an attachment location, which is spaced inboard from the side edge. A landing member can be formed on or secured to the body panel to receive a refastenable portion of the fastening member. One or more lines of weakness can be provided along the front or rear body panel such that one or both of the body panels are breakable. The lines of weakness can comprise a perforation or other series of cuts, a thinning, breakage or separation of material, or a strip of a different kind of material bridging portions of the body panel that is more easily torn or broken than the other material thereof, which allow a user or the manufacturer to separate portions of the body panel. For example, the undergarment can be broken along the lines of weakness after the garment is applied to a user, or beforehand. The fastening members are secured to the garment-side surface of the body panel.

It should be understood that, in other embodiments, the fastening members can be secured to the rear body panel and engage the front body panel or, conversely, can be secured to the front body panel and engage the rear body panel, for example along at least a portion that is not elasticized. The fastening members are fixedly secured to the outer, garment-side surface of the front and/or rear body panels, and releasably engage the outer, garment-side surface of the front and/or rear body panels, although it should be understood that the fastening members could be fixedly secured to an inner body-side surface of front and/or rear body panels and releasably engage an inner, body-side surface of the front and/or rear body panels.

When incorporated into a disposable absorbent undergarment, the fastening members include a refastenable portion, such as an array of hook members, adhesives, such as pressure sensitive adhesives, buttons, zippers, snaps and other releasable and reattachable fastening devices. In various embodiments, the fastening member includes one, two or more than two tab members. In one embodiment, the fastening members comprise a carrier member, which is fixedly secured to the side portions of the front body panel with adhesive bonds, sonic bonds, thermal bonds, pinning, stitching or other known types of attachment. In alternative embodiments, the fastening members can be fixedly secured to the rear body panel or to one or both of the front and rear body panels, for example, at the seam, as explained above.

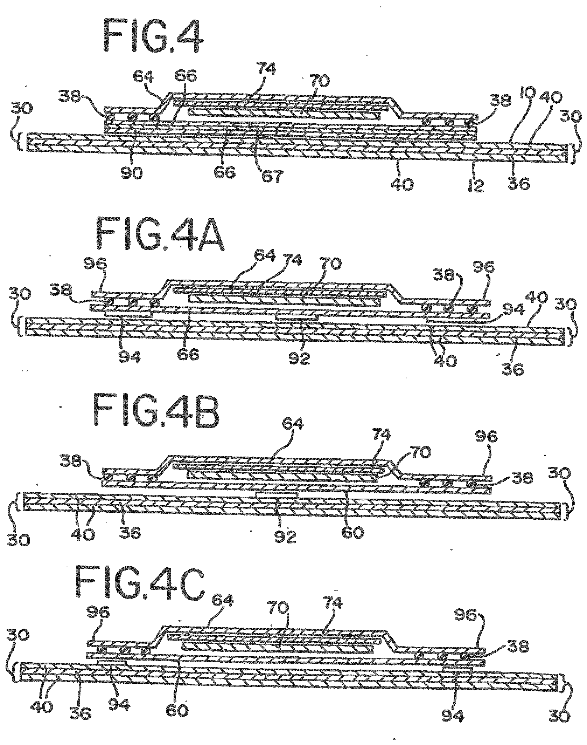

Referring to FIGS. 1-4C, the undergarment is disposable and is also configured as an absorbent undergarment. The crotch member is configured as an absorbent insert **50** having first and second opposed terminal end edges **60**, **62**. The absorbent insert **50** includes a substantially liquid permeable topsheet **64**, or liner, and a substantially liquid impermeable backsheet **66**. A retention portion **70** is disposed or sandwiched between the topsheet and the backsheet, which are connected. In one embodiment, shown in FIGS. 2 and 4, an outer cover **67**, such as a non-woven material, is secured to the backsheet **66**. In other embodiments, shown for example in FIGS. 4A-4C, the outer cover is omitted. In yet another embodiment, the outer cover extends the length of the garment and is disposed over the body panels and crotch member. In yet another embodiment, the outer cover extends the length of and is secured only to the portion of the crotch member that is exposed between and bridges the front and rear body panels. In yet another embodiment, the outer cover, which extends the length of the garment, makes up one of the layers of the front and rear body panels. In various embodiments, the outer cover is made of the same material as the body panels, including for example a non-woven material.

The topsheet **64**, backsheet **66** and other components of the absorbent insert **50** can be joined for example with adhesive bonds, sonic bonds, thermal bonds, pinning, stitching or any other attachment techniques known in the art, as well as combinations thereof. For example, a uniform continuous layer of adhesive, a patterned layer of adhesive, a sprayed pattern of adhesive or any array of lines, swirls or spots of construction bonds may be used to join the topsheet and backsheet, or any of the other components described herein. One or more crotch elastic members **38** are sandwiched between the top sheet **64** and backsheet **66**. It should be understood that the term "absorbent insert" refers to any material or assembly capable of absorbing liquid or bodily exudates, and may be comprised of a single material or component, for example a retention portion, or can be formed as a composite of several components. It should also be understood that the term "crotch member" refers to any member made of any material, including for example and without limitation those described herein with respect to the body panels and absorbent inserts, and is not limited to absorbent inserts and/or materials. For example, the crotch member may be made of one or more layers of a non-woven material.

In one embodiment, additional layers, including for example, a intake/distribution layer **72**, otherwise referred to as a surge layer or transfer layer, are also incorporated into the absorbent insert. The surge layer does not run the entire length of the absorbent insert and is shorter than the retention portion. The topsheet can be indirectly joined to the backsheet by affixing the topsheet to intermediate layers, such as the surge layer or retention portion, which in turn is affixed to the backsheet. The absorbent insert also may include barrier cuffs, or leakage control shields, formed along the opposite longitudinally extending edges of the absorbent composite.

The backsheet is liquid impermeable. In one embodiment, the backsheet can be made from a thin plastic film, or other flexible, substantially liquid-impermeable material. As used herein, the term "flexible" means a material that is compliant and which will readily conform to the general shape and contour of the body of the user. The backsheet prevents various bodily fluids and exudates from wetting or otherwise contaminating various bedding or outer garments worn by the user over the absorbent garment. In particular, the backsheet can include a film, such as a polyethylene film, having a thickness of from about 0.012 mm to about 0.051 mm.

In various constructions, the topsheet can comprise various woven or nonwoven materials. For example, the topsheet can be composed of a meltblown or spunbonded web of desired fibers, and may also be a bonded-carded web. For example, the topsheet can be made of a substantially hydrophobic material, and the hydrophobic material may optionally be treated with a surfactant or otherwise processed to import a desired level of wettability and hydrophilicity. In one particular embodiment of the invention, the topsheet is a nonwoven, spunbond polypropylene fabric composed of about 2.8 - 3.2 denier fibers formed into a web having a basis weight of about 22 gsm and density of about 0.06 gm/cc. The fabric can be surface treated with an operative amount of surfactant, such as about 0.28% Triton X-102 surfactant. The surfactant can be applied by any conventional means, such as spraying, printing, brush coating or the like.

In various constructions, the backsheet can comprise a woven or nonwoven fibrous web layer, which is treated or constructed, partially or wholly, to impart the desired levels of liquid impermeability to selected regions that are adjacent to or proximate the absorbent retention portion. For example, the backsheet may include a gas-permeable, nonwoven fabric layer laminated to a polymer film layer which may or may not be gas-permeable. Other examples of fibrous, cloth-like backsheet materials can comprise a stretch thinned or stretch thermal laminate material composed of a 0.6 mil (0.015 mm) thick polypropylene cast film and a 0.7 ounce per square yard (23.8 gsm) polypropylene spunbond material (2 denier fibers). A material of this type has been employed to form the outercover of a HUGGIES® Ultratrim Disposable Diaper, which has been commercially available from Kimberly-Clark Corporation. The backsheet can provide the outercover of the article, particularly in the crotch region. Optionally, however, the article may include a separate outercover component member, as disclosed herein, which is additional to the backsheet. The outercover can be joined, for example, to one or more of the absorbent composite and/or body panels as explained above.

The backsheet may include a micro-porous, "breathable" material which permits gases, such as water vapor, to escape from the absorbent garment while substantially preventing liquid exudates from passing through the backsheet. For example, the breathable backsheet may be composed of a microporous polymer film or a nonwoven fabric which has been coated or otherwise modified to impart a desired level of liquid impermeability. For example, a suitable microporous film can be a PMP-1 material, which is available from Mitsui Toatsu Chemicals, Inc., a company having offices in Tokyo, Japan; or an XKO-8044 polyolefin film available from 3M Company of Minneapolis, Minnesota. The backsheet may also be embossed or otherwise provided with a pattern or matte finish to exhibit a more aesthetically pleasing appearance.

In various configurations of the invention, where a component, such as the backsheet is configured to be permeable to gas while having a resistance and limited permeability to aqueous liquid, the liquid resistant component can have a construction which is capable of supporting a selected hydrohead of water substantially without leakage therethrough. A suitable technique for determining the resistance of a material to liquid penetration is Federal Test Method Standard FTMS 191 Method 5514, 1978, or an equivalent thereof.

In one embodiment, the backsheet is sufficiently impermeable to liquid and semi-liquid materials to substantially prevent the undesired leakage of waste materials, defined as exudates, including for example urine and feces. For example, the backsheet member can desirably support a hydrohead of at least about 45 centimeters (cm) substantially without leakage. The backsheet member can alternatively support a hydrohead of at least about 55 cm, and optionally, can support a hydrohead of at least about 60 cm, or more, to provide improved benefits.

The backsheet and/or outercover also can be extensible. In one embodiment, the backsheet and/or outercover is capable of providing an elongation of at least about I cm when subjected to a tensile force of 11.8 g/cm, and further provides a substantially permanent deformation of at least about 20% when subjected to a tensile force of 19.70 g/cm and is then allowed to relax under a zero applied force for a period of I minute.

For example, the extensible member can be composed of a necked fiber, a creped fiber, a micro-pleated fiber, polymer films or the like, as well as combinations thereof. The fabrics may be woven or nonwoven materials, such as spunbond fabrics. One example of a suitable extensible material is a 60% necked, polypropylene spunbond having a basis weight of about 1.2 osy.

The backsheet and/or outercover also can be expandable, for example when it has one or more folds, e.g., one or more z-folds (not shown), or can be both extensible and expandable. The term expandable as used herein means to enlarge or to increase the extent or area, lateral and/or longitudinal, thereof, e.g., by unfolding one or more folds.

The retention portion **70** is made of an absorbent material, which can be any material that tends to swell or expand as it absorbs exudates, including various liquids and/or fluids excreted or exuded by the user. For example, the absorbent material can be made of airformed, airlaid and/or wetlaid composites of fibers and high absorbency materials, referred to as superabsorbents. Superabsorbents typically arc made of polyacrylic acids, such as FAVOR 880 available from Stockhausen, Inc. of Greensboro, North Carolina. The fibers can be fluff pulp materials, such as Dalliance CR-1654, or any combination of crosslinked pulps, hardwood, softwood, and synthetic fibers. Airlaid and wetlaid structures typically include binding agents, which are used to stabilize the structure. In addition, various foams, absorbent films, and superabsorbent fabrics can be used as an absorbent material. Various acceptable absorbent materials are disclosed in U.S. Patents 5,147,343 for Absorbent Products Containing Hydrogels With Ability To Swell Against Pressure, 5,601,542 for Absorbent Composite, and 5,651,862 for Wet Formed Absorbent Composite.
Furthermore, the proportion of high-absorbency particles can range from about 0 to about 100%, and the proportion of fibrous material from about 0 to about 100%. Additionally, high absorbency fibers can be used such as Oasis type 121 and type 122 superabsorbent fibers available from Technical Absorbent Ltd., Grimsby, Lincolnshire, United Kingdom.

The retention portion can be made of a single or dual layer of absorbent material. The retention portion has an how-glass shape with enlarged end regions. Alternatively, the retention portion can include a folded or multi-layered configuration. The retention portion has a length substantially equal to, or slightly shorter than, the length of the absorbent insert. The retention portion can include one or more barrier layers attached to the absorbent material. In one embodiment, an upper tissue substrate is disposed adjacent the retention portion. Alternatively, a lower tissue substrate **74** can be disposed adjacent an opposite side of the retention portion, or the tissue can completely envelope the retention position.

Referring to FIGS. 1 and 4-4C, the opposite garment side of the end regions of the crotch member **50**, and in particular, the outer, garment side surface of the backsheet, are secured to the bodyside surface of the opposed crotch portions **16**, **24** of the first and second body panels **4**, **6**. It should be understood that in an alternative embodiment, the crotch member, for example the body side thereof, can be secured to the garment side surface of the first and second body panels. It should be understood that the crotch member **50** can be secured using any of the methods of attachment described above, including for example various adhesives, stitching or other bonding methods. The crotch member can be secured to the body panels with any configuration of attachment lines, swirls, patterns, spots, etc., or can be a full and continuous attachment therebetween.

The entire portion of the crotch member **50** overlapping the body panels **4**, **6** can be attached thereto, as shown in FIG. 4, with a continuous attachment **90**. It should be understood that the various attachment configurations shown in FIGS. 4-4C, which illustrate the crotch member and front body panel, apply equally to the attachment configuration between the rear body panel and the crotch member. As shown in FIG. 4B, the crotch member **50** can be minimally attached to the body panels **4**, **6**, for example, by one or more lines of attachment **92** formed along the centerline of the absorbent insert **50**. In yet another alternative embodiment, shown in FIG. 4C, the crotch member is attached to the body panels **4**, **6** along outer longitudinal edges of the crotch member at attachment locations **94**. In the embodiment of FIG. 4A, the crotch member **50** is attached along the outer edges thereof at locations **94** and also along a location **92**, for example the centerline, therebetween. As shown in FIGS. 4A-4C, the outer lines of attachment **94** are spaced inboard of the outer edge of the crotch member **50** so as to form an unattached side margin **96**, which functions as a flap while maximizing the ability of the underlying body panel to elongate and conform to the body of the user. The attachment locations **92** and **94** run the entirety of the length of the portion of the crotch member overlapping the corresponding body panel, although it should be understood that the attachment locations could be less than such a length, or comprise intermittent portions of attachment along each attachment location or line.

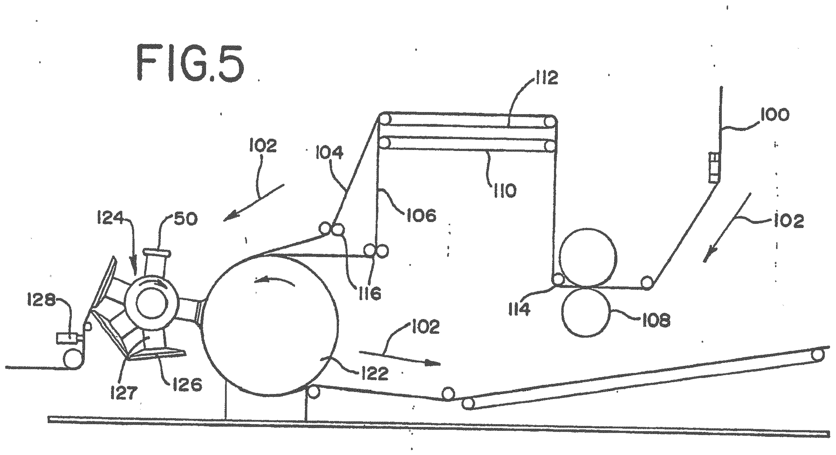

Referring to FIG. 5, a method and apparatus for fabricating one or more embodiments of the aforedescribed refastenable garments is illustrated. Referring to FIG. 5, one or more webs **100**, **104**, **106** of body panel material are moved in a longitudinal machine direction **102**. The body panel material is elongated in the longitudinal machine direction to an elongated condition, for example by stretching the body panel material. In another embodiment, the body panel material is elongated in the lateral cross-direction. The web is elongated between about 50% and about 250% in the longitudinal machine direction.

After the body panel material(s) **104**, **106** are elongated in the longitudinal machine direction **102**, the crotch member **50** is secured to the body panel material webs **104**, **106** in one or more the attachment configurations and at the various attachment locations described herein, with the attachment locations extending in a lateral cross direction and being spaced apart in the longitudinal machine direction. The body panel material is then allowed to retract in the longitudinal machine direction. The retraction of the body panel material gathers the crotch **50** member in the longitudinal machine direction, especially when the crotch member is attached to the body panel material as shown in one of FIGS. 4, 4B and 4C. Alternatively, if the body panel material is elongated in the lateral cross direction, the crotch member is attached to thereto along one or more longitudinally extending locations that are laterally spaced in the cross direction, such the crotch member is gathered in the lateral cross direction.

In various embodiments, the method includes elongating front and rear body panel materials that are integral, or are formed at least in part from one piece. For example, as explained above, an outer cover can extend the length of the garment and can form one layer of each of the front and rear body panels. In another embodiment, a one-piece body chassis forms the entire front and rear body panels and a connecting crotch portion. In other embodiments, the front and rear body panels are discrete members and are separate and spaced apart in the lateral cross direction. The front and rear body panel materials, whether discrete or integral, can be elongated the same or different amounts in the percentage ranges disclosed above. The crotch member has opposite end portions attached to both of the body panel materials, as explained above, when they are in the elongated condition. The body panel materials are then allowed to retract or relax so as to thereby gather the crotch member in the direction of the elongation at both ends thereof.

Referring to FIG. 5, a single web **102** of body panel material can be cut in the longitudinal machine direction to form the front and rear body panel material web **104**, **106**. In one embodiment, the web **102** is cut in a sinusoidal wave pattern, which should be broadly interpreted as a pattern having peaks and valleys, with a cutter **108**, which can be configured as a pair of drums, for example a knife and anvil roll. In various embodiments, the cut edges can be formed by an oscillating cutter, slitters, water jets, lasers and other known cutting devices. The pattern can be formed of undulating curves or wave patterns, or can include or be made entirely of various linear portions.

Each body panel web **102**, **104** includes an outer lateral edge and an inner cut edge. In one embodiment, the inner cut edges of the front and rear body panel webs correspond, or mate such that they have the same shape and amplitude. In such an embodiment, no waste material is generated.

In one embodiment, one or both of the front and rear body panel material webs are shifted in the longitudinal machine direction **102**, as shown to align the crotch portions of the body panel material webs. For example, as shown in FIG. 5, a first and second pivot conveyor **110**, **112** can simply be spaced apart so as to provide for a longer travel for one of the front and rear body panel webs **104**, **106**. In various embodiments, the spacing between the cut edges at the maximum rise of the respective front and rear body panels (i.e., the closest spacing between the front and rear body panels) in one embodiment is between about 1% and about 90% of the total rise of the garment, in another embodiment between about 10% and about 60% of the total rise (or length) of the garment, and in another embodiment between about 20% and about 40% of the total rise of the garment. In addition, in various embodiments, the spacing between the cut edges at the maximum rise of the respective front and rear body panels is between about 10 mm and about 800 mm, between about 50 mm and about 500 mm, and between about 100 mm and about 300 mm. In an alternative embodiment, the cut edges and the crotch portions of the front and rear body panels overlap, and can be secured one to the other. In such an embodiment, the panels can be separated slightly, or can simply be shifted in the longitudinal direction without any lateral separation.

In one embodiment, the front and rear body panel webs **104**, **106** are separated such that no portions of either web overlap each other. For example, and referring to FIG. 5, in one embodiment, pivot conveyors **110**, **112** can be employed to spread the webs. In one embodiment, a first pair of rollers **114** can be angled or twisted to laterally spread the front and rear body panel webs **104**, **106** a first amount before they are shifted in the longitudinal machine direction. A plurality of pairs of rollers **116** can be angled or twisted to laterally spread the front and rear body panel webs **104**, **106** a second amount after they are shifted in the longitudinal machine direction. Of course, it should be understood that the front and rear body panels can be first shifted in the longitudinal machine direction the desired amount and then separated in the lateral cross direction the entire desired amount, or they can also be first separated in the lateral cross direction the entire desired amount and then shifted in the longitudinal machine direction. The body panel webs **104**, **106** are put in tension and are elongated by the pairs of nip rollers **116**. It should be understood that the webs can be put in tension at any number of different locations in the processing line. In addition, the front and rear body panels webs can be subject to different amounts of tension.

It should be understood that webs **104**, **106** having an undulating cross-direction length, or a non-linear terminal crotch edge, will have a tension gradient formed across the length thereof in the cross direction at the peaks **118**, **120**. In other words, the machine direction tension exerted on the web is not uniform between the terminal edges **22**, **28**, **20**, **14**, and will drop off due to the discontinuity of the web in the machine direction. In this way, the body panel webs are not gathered as much, or at all, in the crotch region of the body panels after the body panels are relaxed with the crotch member secured thereto. Moreover, if the crotch member is secured to the body panel along at least a portion of the tension gradient, the body panel will be gathered lesser amounts in the area of lesser tension (and resultant elongation) upon relaxation of the body panel material. In this way, the crotch member will have a gathering gradient, with the crotch member being gathered in the lateral direction lesser amounts along its length.

As shown in FIG. 1, the crotch member **50**, at least the end portions thereof, is secured to the body panels **4**, **6** in an area where the body panel materials are in tension. In other embodiments, the elastic can be deadened in various selected regions to further control and limit the amount of tension and corresponding stretch and subsequent gathering of the body panel webs and body panels.

After the body panel webs **104**, **106** are aligned and separated, regardless of the order thereof, a plurality of crotch members **50**, for example absorbent inserts, are positioned in the lateral cross direction so as to bridge the gaps between the body panel webs at successive peaks **118**, **120** (shown in FIG. 1) where the maximum rises of the body panel webs are aligned. It should be understood that the term "gap" as used herein includes a "zero" distance between the respective cut edges, wherein the cut edges abut but do not overlap. The crotch members **50** are secured to the body panel webs **104**, **106** as explained above. It should be understood that the crotch members **50** are secured to a bodyside surface of the body panel webs, although they can also be secured to the garment side thereof. In one embodiment, the crotch members, for example the absorbent inserts, are assembled offline and are then applied to the front and rear body panel webs **76**, **78** as those webs are carried by a construction drum **122**.

As shown in FIG. 5, the crotch member is rotated using an offset cam action rotator **124**. The rotator includes a plurality of transfer segments **126**, which can have a vacuum applied thereto, that engage the crotch member **50**. Coupler arms **127** connect the transfer segments and a drive ring. The coupler arm **127** includes a cam end having a cam follower that follows the profile of a cam mechanism. The profile of the cam mechanism can be readily changed to change the desired speed output and pitch of the crotch member. In one embodiment, the rotator is configured to accelerate the crotch member. If the successive crotch members **50** are separated by a perforation, the transfer segment **126** breaks the perforation as it engages one crotch member and moves away from the next crotch member, which is engaged by a next transfer segment **126**. The rotator rotates the end portion of the transfer segment, for example approximately 90 degrees, about a radial axis, such that the crotch member is oriented in the machine direction as described above as the transfer segments are rotated about a horizontal axis **128**. Alternatively, the landing material and fastener material are cut and separated by the transfer segments. The rotator, and the method for the use thereof, is further disclosed in U.S. Patent Nos. 5,761,478, 5,759,340, and 6,139,004, and U.S Patent Application S/N 10/038,766, entitled "Apparatus For Applying Discrete Parts to A Moving Web," filed Jan 2, 2002, all of which are assigned to Kimberly-Clark Worldwide, Inc., the assignee of the present application.
Alternatively, the subassembly can be rotated using a revolving transfer roll as shown and described in U.S. Patent No. 4,608,115, which is assigned to Kimberly-Clark Worldwide, Inc., the assignee of the present application. In one embodiment, adhesive is applied to the crotch member **50** by an adhesive applicator **128** prior to the successive separation thereof by the rotator **124**.

Referring to FIG. 5, after the crotch members **50** are secured to the elongated body panel material webs **104**, **106** across the gap, the undergarments, and in particular the crotch members **50**, are successively folded, using for example a helical folder, such that the front and rear body panel webs are positioned in an overlapping, or overlying relationship, for example with the outer edges aligned. In various alternative embodiments, the body panel webs can be secured, for example by bonding using a side seam bonder, to form the side seam. The front and rear body panel webs are then cut along the lateral cross direction along the seam to form a plurality of discrete disposable undergarments, each having a front and rear body panel **4**, **6**. As the body panel webs **104**, **106** are cut, they are allowed to relax, thereby allowing the webs to retract and gather the crotch member **50**. Alternatively, the front and rear body panel webs can be first cut, and the crotch member **50** thereafter folded. In yet another embodiment, the webs can be allowed to relax before the side seams are bonded.

Various refastenable fastening members can be applied to the front and rear body panels **4**, **6** or front and rear body panel webs before or after the various cutting and folding operations. In one embodiment, as explained above, the undergarment can be configured with side seams which secure the front and rear body panels, and refastenable fastening members, which bridge lines of weakness formed in one or the other of the body panels.

Although the present invention has been described with reference to various exemplary embodiments, those skilled in the art will recognize that changes may be made in form and detail without departing from the scope of the invention. As such, it is intended that the foregoing detailed description be regarded as illustrative rather than limiting and that it is the appended claims, including all equivalents thereof, which are intended to define the scope of the invention.

## Claims

1. A method of manufacturing a disposable undergarment comprising:
elongating a body panel material (4, 6) in a first direction to an elongated condition, wherein said body panel material (4, 6) is a laminate material with one or more elastic elements (36) sandwiched therebetween;
attaching a crotch member (50) to said body panel material (4, 6) while said body panel material (4, 6) is in said elongated condition; and
retracting said body panel material (4, 6) in said first direction from said elongated condition to a relaxed condition;
**characterized in that:**
said crotch member (50) is configured as an absorbent insert (50) having first and second opposed terminal end edges (60, 62) and comprising a substantially liquid permeable topsheet (64) connected to a substantially liquid impermeable backsheet (66) and a retention portion (70) disposed between said topsheet (64) and said backsheet (66);
wherein said absorbent insert (50) is attached to said body panel material (4, 6) while said body panel material (4, 6) is in said elongated condition; and retracting said body panel material (4, 6) comprises gathering said absorbent insert (50) in said first direction;
wherein said elongating said body panel material (4, 6) in said first direction to said elongated condition comprises elongating said body panel material (4, 6) between about 50% and about 250% in said first direction.

2. The method of claim 1 wherein said body panel material (4, 6) comprises a front body panel material (4) and wherein said elongated and relaxed conditions comprise first elongated and relaxed conditions respectively, and further comprising a rear body panel material (6) spaced apart from said front body panel material (4) in a second direction, and further comprising elongating said rear body panel material (6) in said first direction to a second elongated condition, attaching said crotch member (50) to said rear body panel material (6) while said rear body panel material (6) is in said second elongated condition, and retracting said rear body panel material (6) in said first direction from said second elongated condition to a second relaxed condition.

3. The method of claim 2 wherein said retracting said rear body panel material (6) comprises gathering said crotch member (50) in said first direction.

4. The method of claim 1 wherein said crotch member (50) has a length, opposite end portions and opposite side edges, wherein at least one of said opposite end portions overlies said body panel material (4, 6), and wherein said attaching said crotch member (50) to said body panel material (4, 6) comprises attaching said end portion of said crotch member (50) overlying said body panel material (4, 6) to said body panel material (4, 6).

5. The method of claim 4 wherein said attaching said end portion of said crotch member (50) to said body panel material (4, 6) comprises attaching said end portion to said body panel material (4, 6) at a plurality of attachment locations (92, 94) spaced apart in said first direction.

6. The method of claim 5 wherein said plurality of attachment locations (92, 94) comprises at least a pair of attachment locations (94) positioned adjacent said opposite side edges of said crotch member (50).

7. The method of claim 6 wherein said at least said pair of attachment locations (94) run substantially an entirety of said end portion that overlies said crotch member (50).

8. The method of claim 5 wherein said plurality of attachment locations (92, 94) comprises at least a pair of attachment locations positioned inboard from said opposite side edges of said crotch member (50), wherein said opposite side edges form opposite unattached flaps overlying said body panel material (4,6).

9. The method of claim 4 wherein said attaching said end portion of said crotch member (50) to said body panel material (4, 6) comprises attaching said end portion to said body panel material (4, 6) at an attachment location (92) located substantially along a centerline of said crotch member (50).

10. The method of claim 9 wherein said attachment location (92) has a width less than a width of said crotch member (50) measured between said opposite sides thereof.

11. The method of any preceding claim wherein said attaching said crotch member (50) to said body panel material (4, 6) comprises attaching said crotch member (50) to a bodyside surface of said body panel material (4, 6).

12. The method of claim 1 wherein said attaching said crotch member (50) to said body panel material (4, 6) comprises attaching said crotch member (50) to a garment side surface of said body panel material (4, 6).

13. The method of claim 1, wherein said body panel material (4, 6) is a first body panel material (4) elongated in a first direction to a first elongated condition; further comprising:
elongating a second body panel material (6) in said first direction to a second elongated condition;
attaching said absorbent insert (50) to said first (4) and second (6) body panel materials at a plurality of attachment locations (92, 94) while said first (4) and second (6) body panel materials are in said first and second elongated conditions, wherein said attachment locations (92, 94) are spaced apart in said first direction; and
retracting said first (4) and second (6) body panel materials in said first direction from said first and second elongated conditions to first and second relaxed conditions respectively.

14. The method of claim 13 wherein said retracting said first (4) and second (6) body panel materials comprises gathering said absorbent insert (50) in said first direction.

15. The method of claim 13 wherein said absorbent insert (50) has a length, first and second end portions and opposite side edges, wherein said first and second end portions overlie respectively said first (4) and second (6) body panel materials, and wherein said attaching said absorbent insert (50) to said first (4) and second (6) body panel materials comprises attaching said first end portion to said first body panel material (4) at a plurality of first attachment locations (92, 94) spaced apart in said first direction and attaching said second end portion to said second body panel material (6) at a plurality of second attachment locations (92, 94) spaced apart in said first direction.

16. The method of claim 15 wherein said pluralities of first and second attachment locations (92, 94) comprises at least a pair of said first attachment locations (92, 94) positioned inboard from said opposite side edges of absorbent insert (50) and at least a pair of said second attachment locations (92, 94) positioned inboard from said opposite side edges of absorbent insert (50), wherein said opposite side edges form opposite unattached flaps overlying said first (4) and second (6) body panel materials.

17. The method of claim 15 wherein said elongating said first (4) and second (6) body panel materials in said first direction to said first and second elongated conditions respectively comprises elongating said first (4) and second (6) body panel materials at least about 10% in said first direction.

18. The method of claim 17 wherein said elongating said first (4) and second (6) body panel materials at least 10% in said first direction comprises elongating said first (4) and second (6) body panel materials between about 25% and about 250% in said first direction.

19. The method of claim 18 wherein said elongating said first (4) and second (6) body panel materials between about 25% and about 250% in said first direction comprises elongating said first (4) and second (6) body panel materials between about 50% and about 150% in said first direction.

## Patentansprüche

1. Verfahren zur Herstellung einer Einwegunterwäsche, Folgendes umfassend:
Strecken eines Körperbahnmaterials (4, 6) in eine erste Richtung zu einem gestreckten Zustand, wobei das Körperbahnmaterial (4, 6) ein Laminatmaterial mit einem oder mehreren dazwischen sandwichartig angeordneten elastischen Elementen (36) ist;
Befestigen eines Schrittelements (50) an dem Körperbahnmaterial (4, 6), während sich das Körperbahnmaterial (4, 6) in dem gestreckten Zustand befindet; und
Zurückziehen des Körperbahnmaterials (4, 6) in die erste Richtung von dem gestreckten Zustand zu einem entspannten Zustand;
**dadurch gekennzeichnet, dass:**
das Schrittelement (50) als eine absorptionsfähige Einlage (50) ausgeführt ist, die erste und zweite gegenüberliegende terminale Endränder (60, 62) aufweist und eine im Wesentlichen flüssigkeitsdurchlässige Oberschicht (64), die mit einer im Wesentlichen flüssigkeitsundurchlässigen Hinterschicht (66) verbunden ist, und einen Rückhalteabschnitt (70) umfasst, der zwischen der Oberschicht (64) und der Hinterschicht (66) angeordnet ist;
wobei die absorptionsfähige Einlage (50) an dem Körperbahnmaterial (4, 6) befestigt ist, während sich das Körperbahnmaterial (4, 6) in dem gestreckten Zustand befindet; und
wobei das Zurückziehen des Körperbahnmaterials (4, 6) das Raffen der absorptionsfähigen Einlage (50) in die erste Richtung umfasst;
wobei das Strecken des Körperbahnmaterials (4, 6) in die erste Richtung zu dem gestreckten Zustand das Strecken des Körperbahnmaterials (4, 6) zwischen etwa 50 % und etwa 250 % in die erste Richtung umfasst.

2. Verfahren nach Anspruch 1, wobei das Körperbahnmaterial (4, 6) ein vorderes Körperbahnmaterial (4) umfasst, und wobei der gestreckte und entspannte Zustand jeweils einen ersten gestreckten und entspannten Zustand umfasst, und des Weiteren ein hinteres Körperbahnmaterial (6) umfasst, das von dem vorderen Körperbahnmaterial (4) in einer zweiten Richtung beabstandet ist, und das des Weiteren das Strecken des hinteren Körperbahnmaterials (6) in die erste Richtung zu einem zweiten gestreckten Zustand umfasst, Befestigen des Schrittelements (50) an dem hinteren Körperbahnmaterial (6), während das hintere Körperbahnmaterial (6) sich in dem zweiten gestreckten Zustand befindet, und Zurückziehen des hinteren Körperbahnmaterials (6) in die erste Richtung von dem zweiten gestreckten Zustand zu einem zweiten entspannten Zustand.

3. Verfahren nach Anspruch 2, wobei das Zurückziehen des hinteren Körperbahnmaterials (6) das Raffen des Schrittelements (50) in die erste Richtung umfasst.

4. Verfahren nach Anspruch 1, wobei das Schrittelement (50) eine Länge, gegenüberliegende Endabschnitte und gegenüberliegende Seitenränder aufweist, wobei mindestens einer der gegenüberliegenden Endabschnitte das Körperbahnmaterial (4, 6) überlagert, und wobei das Befestigen des Schrittelements (50) an dem Körperbahnmaterial (4, 6) das Befestigen des Endabschnitts des Schrittelements (50), das das Körperbahnmaterial (4, 6) überlagert, an dem Körperbahnmaterial (4, 6) umfasst.

5. Verfahren nach Anspruch 4, wobei das Befestigen des Endabschnitts des Schrittelements (50) an dem Körperbahnmaterial (4, 6) das Befestigen des Endabschnitts an dem Körperbahnmaterial (4, 6) an einer Vielfalt von Befestigungsstellen (92, 94) umfasst, die in der ersten Richtung beabstandet sind.

6. Verfahren nach Anspruch 5, wobei die Vielfalt von Befestigungsstellen (92, 94) mindestens ein Paar von Befestigungsstellen (94) umfasst, das angrenzend an die gegenüberliegenden Seitenränder des Schrittelements (50) angeordnet ist.

7. Verfahren nach Anspruch 6, wobei das mindestens eine Paar von Befestigungsstellen (94) im Wesentlichen über die Gesamtheit des Endabschnitts verläuft, der das Schrittelement (50) überlagert.

8. Verfahren nach Anspruch 5, wobei die Vielzahl von Befestigungsstellen (92, 94) mindestens ein Paar von Befestigungsstellen umfasst, die innerseitig von den gegenüberliegenden Seitenrändern des Schrittelements (50) angeordnet sind, wobei die gegenüberliegenden Seitenränder gegenüberliegende nicht befestigten Laschen bilden, die das Körperbahnmaterial (4, 6) überlagern.

9. Verfahren nach Anspruch 4, wobei das Befestigen des Endabschnitts des Schrittelements (50) an dem Körperbahnmaterial (4, 6) das Befestigen des Endabschnitts an dem Körperbahnmaterial (4, 6) an einer Befestigungsstelle (92) umfasst, die im Wesentlichen entlang einer Mittellinie des Schrittelements (50) angeordnet ist.

10. Verfahren nach Anspruch 9, wobei die Befestigungsstelle (92) eine Breite aufweist, die geringer ist als eine Breite des Schrittelements (50), die zwischen den gegenüberliegenden desselben gemessen wird.

11. Verfahren nach einem der vorhergehenden Ansprüche, wobei das Befestigen des Schrittelements (50) an dem Körperbahnmaterial (4, 6) das Befestigen des Schrittelements (50) an eine körperseitige Fläche des Körperbahnmaterials (4, 6) umfasst.

12. Verfahren nach Anspruch 1, wobei das Befestigen des Schrittelements (50) an das Körperbahnmaterial (4, 6) das Befestigen des Schrittelements (50) an eine kleidungsseitige Fläche des Körperbahnmaterials (4, 6) umfasst.

13. Verfahren nach Anspruch 1, wobei das Körperbahnmaterial (4, 6) ein erstes Körperbahnmaterial (4) ist, das in einer ersten Richtung zu einem ersten gestreckten Zustand gestreckt ist; des Weiteren Folgendes umfassend:
das Strecken eines zweiten Körperbahnmaterials (6) in die erste Richtung zu einem zweiten gestreckten Zustand;
Befestigen der absorptionsfähigen Einlage (50) an dem ersten (4) und zweiten (6) Körperbahnmaterial an einer Vielfalt von Befestigungsstellen (92, 94), während das erste (4) und zweite (6) Körperbahnmaterial sich in dem ersten und zweiten gestreckten Zustand befinden, wobei die Befestigungsstellen (92, 94) in der ersten Richtung voneinander beabstandet sind; und
Zurückziehen des ersten (4) und zweiten (6) Körperbahnmaterials in die erste Richtung von dem ersten und zweiten gestreckten Zustand zu jeweils einem ersten und zweiten entspannten Zustand.

14. Verfahren nach Anspruch 13, wobei das Zurückziehen des ersten (4) und zweiten (6) Körperbahnmaterials das Raffen der absorptionsfähige Einlage (50) in die erste Richtung umfasst.

15. Verfahren nach Anspruch 13, wobei die absorptionsfähige Einlage (50) eine Länge, einen ersten und zweiten Endabschnitt und gegenüberliegende Seitenränder aufweist, wobei der erste und zweite Endabschnitt jeweils das erste (4) und zweite (6) Körperbahnmaterial überlagern, und wobei das Befestigen der absorptionsfähigen Einlage (50) an dem ersten (4) und zweiten (6) Körperbahnmaterial das Befestigen des ersten Endabschnitts an dem ersten Körperbahnmaterial (4) an einer Vielfalt von ersten Befestigungsstellen (92, 94), die in der ersten Richtung voneinander beabstandet sind und das Befestigen des zweiten Endabschnitts an dem zweiten Körperbahnmaterial (6) an einer Vielfalt von zweiten Befestigungsstellen (92, 94), die in der ersten Richtung voneinander beabstandet sind, umfasst.

16. Verfahren nach Anspruch 15, wobei die Vielzahlen von ersten und zweiten Befestigungsstellen (92, 94) mindestens ein Paar von den ersten Befestigungsstellen (92, 94) umfasst, das innerseitig von den gegenüberliegenden Seitenrändern der absorptionsfähigen Einlage (50) angeordnet ist, und mindestens ein Paar von den zweiten Befestigungsstellen (92, 94), das innerseitig von den gegenüberliegenden Seitenrändern der absorptionsfähigen Einlage (50) angeordnet ist, wobei die gegenüberliegenden Seitenränder gegenüberliegende nicht befestigte Laschen bilden, die das erste (4) und zweite (6) Körperbahnmaterial überlagern.

17. Verfahren nach Anspruch 15, wobei das Strecken des ersten (4) und zweiten (6) Körperbahnmaterials in die erste Richtung zu dem ersten und zweiten gestreckten Zustand jeweils das Strecken des ersten (4) und zweiten (6) Körperbahnmaterials um mindestens etwa 10 % in die erste Richtung umfasst.

18. Verfahren nach Anspruch 17, wobei das Strecken des ersten (4) und zweiten (6) Körperbahnmaterials um mindestens 10 % in die erste Richtung das Strecken des ersten (4) und zweiten (6) Körperbahnmaterials zwischen etwa 25 % und etwa 250 % in die erste Richtung umfasst.

19. Verfahren nach Anspruch 18, wobei das Strecken des ersten (4) und zweiten (6) Körperbahnmaterials zwischen etwa 25 % und etwa 250 % in die erste Richtung das Strecken des ersten (4) und zweiten (6) Körperbahnmaterials zwischen etwa 50 % und etwa 150 % in die erste Richtung umfasst.

## Revendications

1. Procédé de fabrication d'un sous-vêtement jetable, comprenant :
l'allongement d'un matériau de panneau de corps (4, 6) dans une première direction jusqu'à un état allongé, dans lequel ledit matériau de panneau allongé (4, 6) est un matériau stratifié avec un ou plusieurs éléments élastiques (36) intercalés entre celui-ci ;
la fixation d'un organe d'entrejambe (50) audit matériau de panneau de corps (4, 6) pendant que ledit matériau de panneau de corps (4, 6) est dans ledit état allongé ; et
la rétraction dudit matériau de panneau de corps (4, 6) dans ladite première direction à partir dudit état allongé jusqu'à un état relâché ;
**caractérisé en ce que** :
ledit organe d'entrejambe (50) est configuré en tant qu'insert absorbant (50) possédant des premier et deuxième bords d'extrémité terminaux opposés (60, 62) et comprenant une feuille supérieure (64) sensiblement perméable aux liquides reliée à une feuille arrière (66) sensiblement imperméable aux liquides, ainsi qu'une partie de rétention (70) disposée entre ladite feuille supérieure (64) et ladite feuille arrière (66) ;
ledit insert absorbant (50) étant fixé audit matériau de panneau de corps (4, 6) pendant que ledit matériau de panneau de corps (4, 6) est dans ledit état allongé ; et
la rétraction dudit matériau de panneau de corps (4, 6) comprenant le fronçage dudit insert absorbant (50) dans ladite première direction ;
dans lequel ledit allongement dudit matériau de panneau de corps (4, 6) dans ladite première direction jusqu'audit état allongé comprend l'allongement dudit matériau de panneau de corps (4, 6) d'entre environ 50 % et environ 250 % dans ladite première direction.

2. Procédé selon la revendication 1, dans lequel ledit matériau de panneau de corps (4, 6) comprend un matériau de panneau de corps avant (4), et dans lequel lesdits états allongé et relâché comprennent des premiers états allongé et relâché respectivement, et comprenant en outre un matériau de panneau de corps arrière (6) espacé dudit matériau de panneau de corps avant (4) dans une deuxième direction, et comprenant en outre l'allongement dudit matériau de panneau de corps arrière (6) dans ladite première direction jusqu'à un deuxième état allongé, la fixation dudit élément d'entrejambe (50) audit matériau de panneau de corps arrière (6) pendant que ledit matériau. de panneau de corps arrière (6) est dans ledit deuxième état allongé, et la rétraction dudit matériau de panneau de corps arrière (6) dans ladite première direction à partir dudit deuxième état allongé jusqu'à un deuxième état relâché.

3. Procédé selon la revendication 2, dans lequel ladite rétraction dudit matériau de panneau de corps arrière (6) comprend le fronçage dudit organe d'entrejambe (50) dans ladite première direction.

4. Procédé selon la revendication 1, dans lequel ledit organe d'entrejambe (50) possède une longueur, des parties d'extrémité opposées et des bords latéraux opposés, dans lequel au moins l'une desdites parties d'extrémité opposées recouvre ledit matériau de panneau de corps (4, 6), et dans lequel ladite fixation dudit organe d'entrejambe (50) audit matériau de panneau de corps (4, 6) comprend la fixation de ladite partie d'extrémité dudit organe d'entrejambe (50) recouvrant ledit matériau de panneau de corps (4, 6) audit matériau de panneau de corps (4, 6).

5. Procédé salon la revendication 4, dans lequel ladite fixation de ladite partie d'extrémité dudit organe d'entrejambe (50) audit matériau de panneau de corps (4, 6) comprend la fixation de ladite partie d'extrémité audit matériau de panneau de corps (4, 6) en une pluralité d'emplacements de fixation (92, 94) espacés dans ladite première direction.

6. Procédé selon la revendication 5, dans lequel ladite pluralité d'emplacements de fixation (92, 94) comprend au moins une paire d'emplacements de fixation (94) positionnés de manière adjacente auxdits bords latéraux opposés dudit organe d'entrejambe (50).

7. Procédé selon la revendication 6, dans lequel ladite au moins une paire d'emplacements de fixation (94) s'étend sensiblement sur toute ladite partie d'extrémité qui recouvre ledit organe d'entrejambe (50).

8. Procédé selon la revendication 5, dans lequel ladite pluralité d'emplacements de fixation (92, 94) comprend au moins une paire d'emplacements de fixation positionnés à l'intérieur par rapport auxdits bords latéraux opposés dudit organe d'entrejambe (50), dans lequel lesdits bords latéraux opposés forment des rabats non fixés opposés recouvrant ledit matériau de panneau de corps (4, 6).

9. Procédé selon la revendication 4, dans lequel ladite fixation de ladite partie d'extrémité dudit organe d'entrejambe (50) audit matériau de panneau de corps (4, 6) comprend la fixation de ladite partie d'extrémité audit matériau de panneau de corps (4, 6) en un emplacement de fixation (92) situé sensiblement le long d'une ligne médiane dudit organe d'entrejambe (50).

10. Procédé selon la revendication 9, dans lequel ledit emplacement de fixation (92) possède une largeur qui est inférieure à une largeur dudit organe d'entrejambe (50) mesurée entre lesdits côtés opposés de ce dernier.

11. Procédé selon l'une quelconque des revendications précédentes, dans lequel ladite fixation dudit organe d'entrejambe (50) audit matériau de panneau de corps (4, 6) comprend la fixation dudit organe d'entrejambe (50) à une surface côté corps dudit matériau de panneau de corps (4, 6).

12. Procédé selon la revendication 1, dans lequel ladite fixation dudit organe d'entrejambe (50) audit matériau de panneau de corps (4, 6) comprend la fixation dudit organe d'entrejambe (50) à une surface côté vêtement dudit matériau de panneau de corps (4, 6).

13. Procédé selon la revendication 1, dans lequel ledit matériau de panneau de corps (4, 6) est un premier matériau de panneau de corps (4) allongé dans une première direction jusqu'à un premier état allongé ; comprenant en outre :
l'allongement d'un deuxième matériau de panneau de corps (6) dans ladite première direction jusqu'à un deuxième état allongé ;
la fixation dudit insert absorbant (50) auxdits premier (4) et deuxième (6) matériaux de panneau de corps en une pluralité d'emplacements de fixation (92, 94) pendant que lesdits premier (4) et deuxième (6) matériaux de panneau de corps sont dans lesdits premier et deuxième états allongés, dans lequel lesdits emplacements de fixation (92, 94) sont espacés dans ladite première direction ; et
la rétraction desdits premier (4) et deuxième (6) matériaux de panneau de corps dans ladite première direction à partir desdits premier et deuxième états allongés jusqu'à des premier et deuxième états relâchés respectivement.

14. Procédé selon la revendication 13, dans lequel ladite rétraction desdits premier (4) et deuxième (6) matériaux de panneau de corps comprend le fronçage dudit insert absorbant (50) dans ladite première direction.

15. Procédé selon la revendication 13, dans lequel ledit insert absorbant (50) possède une longueur, des première et deuxième parties d'extrémité et des bords latéraux opposés, dans lequel lesdites première et deuxième parties d'extrémité recouvrent respectivement lesdits premier (4) et deuxième (6) matériaux de panneau de corps, et dans lequel ladite fixation dudit insert absorbant (50) auxdits premier (4) et deuxième (6) matériaux de panneau de corps comprend la fixation de ladite première partie d'extrémité audit premier matériau de panneau de corps (4) en une pluralité de premiers emplacements de fixation (92, 94) espacés dans ladite première direction et la fixation de ladite deuxième partie d'extrémité audit deuxième matériau de panneau de corps (6) en une pluralité de deuxièmes emplacements de fixation (92, 94) espacés dans ladite première direction.

16. Procédé selon la revendication 15, dans lequel lesdites pluralités de premiers et deuxièmes emplacements de fixation (92, 94) comprennent au moins une paire desdits premiers emplacements de fixation (92, 94) positionnés a l'intérieur par rapport auxdits bords latéraux opposés de l'insert absorbant (50) et au moins une paire desdits deuxièmes emplacements de fixation (92, 94) positionnés a l'intérieur par rapport auxdits bords latéraux opposés de l'insert absorbant (50), lesdits bords latéraux opposés formant des rabats non fixés opposés recouvrant lesdits premier (4) et deuxième (6) matériaux de panneau de corps.

17. Procédé selon la revendication 15, dans lequel ledit allongement desdits premier (4) et deuxième (6) matériaux de panneau de corps dans ladite première direction jusqu'auxdits premier et deuxième états allongés respectivement comprend l'allongement desdits premier (4) et deuxième (6) matériaux de panneau de corps d'au moins environ 10 % dans ladite première direction.

18. Procédé selon la revendication 17, dans lequel ledit allongement desdits premier (4) et deuxième (6) matériaux de panneau de corps d'au moins 10 % dans ladite première direction comprend l'allongement desdits premier (4) et deuxième (6) matériaux de panneau de corps d'entre environ 25 % et environ 250 % dans ladite première direction.

19. Procédé selon la revendication 18, dans lequel ledit allongement desdits premier (4) et deuxième (6) matériaux de panneau de corps d'entre environ 25 % et environ 250 % dans ladite première direction comprend l'allongement desdits premier (4) et deuxième (6) matériaux de panneau de corps d'entre environ 50 % et environ 150 % dans ladite première direction.
